# EUROPÄISCHE PATENTSCHRIFT

(11) **EP 0 948 571 B1**
(45) Veröffentlichungstag und Bekanntmachung des Hinweises auf die Patenterteilung: **18.06.2003**
(21) Anmeldenummer: 97918104.7
(22) Anmeldetag: 03.04.1997
(51) Int. Cl.: C09C 1/00, C09D 7/12, C09D 11/00, C08K 3/00, C03C 4/02, C04B 33/14, A61K 7/00

(54) **GLANZPIGMENTE AUF BASIS REDUZIERTER TITANDIOXIDBESCHICHTETER SILICIUMDIOXIDPLÄTTCHEN**
GLOSS PIGMENT BASED ON REDUCED TITANIUM DIOXIDE-COATED SILICON DIOXIDE PLATELETS
PIGMENTS BRILLANTS A BASE DE PLAQUETTES DE DIOXYDE DE SILICIUM RECOUVERTES DE DIOXYDE DE TITANE REDUITES

(30) Priorität: 13.04.1996 DE 19614636
(43) Veröffentlichungstag der Anmeldung: 13.10.1999
(73) Patentinhaber: BASF AKTIENGESELLSCHAFT, 67056 Ludwigshafen (DE)
(72) Erfinder: SCHMID, Raimund, 67435 Neustadt (DE); MRONGA, Norbert, D-69221 Dossenheim (DE)
(86) Internationale Anmeldenummer: EP9701673
(87) Internationale Veröffentlichungsnummer: WO97039065

(56) Entgegenhaltungen:
- EP-A- 0 735 115
- DE-A- 4 441 223
- PROCEEDINGS OF THE XIVTH INTERNATIONAL CONFERENCE IN ORGANIC COATINGS SCIENCE AND TECHNOLOGY, 1988, TECHNOMIC PUBL., LANCASTER/BASEL, XP002035233 P. VAPAAOKSA: "Thixotropic and settling properties of TiO2-silica pigment and their impact on surface coating applications"
- DATABASE WPI Week 8345 Derwent Publications Ltd., London, GB; AN 83-810901 XP002038962 & JP 58 164 653 A (MITSUBISHI METAL) , 29.September 1983

## Beschreibung

Die vorliegende Erfindung betrifft neue Glanzpigmente auf Basis beschichteter Siliciumdioxidplättchen, erhältlich durch Belegung der Siliciumdioxidplättchen mit einer im wesentlichen aus Titandioxid bestehenden Schicht und anschließendes Erhitzen der belegten Plättchen in einer reduzierenden Atmosphäre.

Weiterhin betrifft die Erfindung die Herstellung dieser Glanzpigmente und ihre Verwendung zum Einfärben von Lacken, Druckfarben, Tinten, Kunststoffen, Gläsern, keramischen Produkten und Zubereitungen der dekorativen Kosmetik.

Glanz- oder Effektpigmente werden in vielen Bereichen der Technik eingesetzt, beispielsweise in Automobillacken, in der dekorativen Beschichtung, der Kunststoffeinfärbung, in Anstrich-, Druck-, insbesondere Sicherheitsdruckfarben sowie in der Kosmetik.

Ihre optische Wirkung beruht auf der gerichteten Reflexion von Licht an überwiegend flächig ausgebildeten, zueinander parallel ausgerichteten, metallischen oder stark lichtbrechenden Pigmentteilchen. Je nach Zusammensetzung der Pigmentplättchen erzeugen Interferenz-, Reflexions- und Absorptionsphänomene winkelabhängige Farb- und Helligkeitseindrücke.

Aufgrund ihrer nicht kopierbaren optischen Effekte gewinnen diese Pigmente zunehmende Bedeutung für die Herstellung von fälschungssicheren Wertschriften wie Geldscheinen, Schecks, Scheckkarten, Kreditkarten, Steuermarken, Briefmarken, Bahn- und Flugtickets, Telefonkarten, Lotterielosen, Geschenkzertifikaten, Ausweisen und Identifikationskarten.

Kennzeichnungen, die mit den Effektpigmenten angefertigt wurden, und das Fehlen dieser Kennzeichnungen oder ihre Veränderung, beispielsweise in einer Farbkopie (Verschwinden von Farbflops und Glanzeffekten), sind ohne Hilfsmittel mit bloßem Auge sicher erkennbar und ermöglichen so eine leichte Unterscheidung des Originals von der Kopie.

Glimmerpigmente mit Titandioxidbeschichtung zeigen bekanntermaßen nur sehr schwache Interferenzfarben, die erst bei Applikation auf dunklem Untergrund sichtbar werden. Durch Reduzieren der TiO₂-Schicht kann der Farbeindruck zwar verstärkt werden, jedoch beschränkt sich der Farbflop auf einen Wechsel von der jeweiligen Interferenzfarbe nach unbunt. Außerdem ist insbesondere im blauen Farbtonbereich die zu erreichende Farbstärke nicht für alle Anwendungszwecke zufriedenstellend.

Der Erfindung lag die Aufgabe zugrunde, Glanzpigmente mit guten koloristischen und anwendungstechnischen Eigenschaften bereitzustellen, welche die Nachteile der TiO₂-beschichteten Glimmerpigmente nicht aufweisen.

Demgemäß wurden die eingangs definierten Glanzpigmente gefunden.

Weiterhin wurde das hierdurch definierte Verfahren zu ihrer Herstellung gefunden.

Außerdem wurde die Verwendung der Glanzpigmente zur Einfärbung von Lacken, Druckfarben, Tinten, Kunststoffen, Gläsern, keramischen Produkten und Zubereitungen der dekorativen Kosmetik gefunden.

Die bei den erfindungsgemäßen Glanzpigmenten als Substrat dienenden SiO₂-Plättchen können, wie in der WO-A-93/08237 beschrieben. durch Aufstreichen einer Wasserglaslösung in einer Schichtdicke von etwa 10 µm auf ein umlaufendes Band, Trocknen und anschließendes Waschen des Wasserglasfilms zunächst mit Säure und dann mit Wasser, Ablösen des dabei erhaltenen gelartigen SiO₂-Films und Waschen sowie Mahlen der dabei entstandenen Bruchstücke hergestellt werden.

Die SiO₂-Plättchen haben üblicherweise einen Durchmesser von 1 bis 250 µm, bevorzugt 2 bis 100 µm, und sind in der Regel 0,05 bis 5 µm, insbesondere 0,2 bis 2 µm, dick.

Sie können durch den Einbau anorganischer Farbmittel eingefärbt sein. Unter einem anorganischen Farbmittel sind dabei anorganische Pigmente in möglichst feinteiliger Form, die als Feststoffteilchen in die SiO₂-Matrix eingebaut werden, oder farbige Metallkationen, mit denen die Matrix dotiert wird, zu verstehen. Vorzugsweise sind die SiO₂-Plättchen jedoch nicht eingefärbt.

Die erfindungsgemäßen Glanzpigmente können vorteilhaft nach dem erfindungsgemäßen Herstellungsverfahren durch Belegung der SiO₂-Plättchen mit einer im wesentlichen aus TiO₂ bestehenden Schicht und anschließendes Erhitzen der belegten Plättchen in einer reduzierenden Atmosphäre erhalten werden.

Die Belegung der SiO₂-Plättchen mit Titandioxid kann durch Gasphasenzersetzung verdampfter Titanverbindungen in Gegenwart von Wasserdampf (chemical vapor deposition, CVD) erfolgen. Geeignet sind hierfür z.B. Titantetraalkoholate, bevorzugt Titantetraisopropanolat, und Titantetrahalogenide, insbesondere Titantetrachlorid (DE-A-41 40 900, EP-A-106 235).

Die Gasphasenbeschichtung wird zweckmäßigerweise in einem beheizbaren Wirbelschichtreaktor, wie er beispielsweise in der EP-A-106 235 beschrieben ist, durchgeführt, in dem die SiO₂-Teilchen zunächst mit einem Wirbelgas (z.B. Stickstoff) fluidisiert und auf die für die Zersetzung der jeweiligen Titanverbindung erforderliche Temperatur von in der Regel 100 bis 400°C, insbesondere 200 bis 300°C, erhitzt werden. Die in einem vorgeschalteten Verdampfergefäß unter Verwendung eines Teils des Wirbelgases verdampften Titanverbindungen sowie der zur Zersetzung benötigte Wasserdampf werden dann über getrennte Düsen eingetragen.

Die TiO₂-Schicht kann aber auch naßchemisch durch Hydrolyse von Titanverbindungen wie Titantetrachlorid aufgebracht werden, wobei es sich, wie in der WO-A-93/08237 beschrieben, empfiehlt, zunächst durch Hydrolyse geeigneter Zinn(IV)verbindungen, z.B. Zinntetrachlorid, einen dünnen Zinndioxidfilm abzuscheiden.

Bei der Naßbelegung geht man zweckmäßigerweise so vor, daß man die SiO₂-Plättchen unter starkem Rühren in Wasser suspendiert, die erhaltene Suspension auf etwa 70 bis 90°C erhitzt und auf einen pH-Wert von etwa 1 bis 2 einstellt und zunächst eine saure Zinntetrachloridlösung und dann eine Titantetrachloridlösung unter gleichzeitiger Zugabe einer Base, z.B. Natronlauge, zur Konstanthaltung des pH-Wertes zutropft. Die beschichteten SiO₂-Plättchen werden anschließend abfiltriert, mit Wasser gewaschen, getrocknet und bei etwa 800°C geglüht.

Die Dicke der TiO₂-Schicht, die bis zu etwa 5 Gew.-% weitere farblose Metalloxide wie Zinndioxid enthalten kann, bestimmt im wesentlichen den Farbton und beträgt je nach der gewünschten Interferenzfarbe in der Regel 10 bis 300 nm, vorzugsweise 20 bis 200 nm (geometrische Schichtdicke).

Bei den SiO₂-Substratplättchen mit dünner TiO₂-Beschichtung (geometrische Schichtdicke etwa 10 bis 60 nm, insbesondere 20 bis 40 nm), die eine silberne Interferenzfarbe zeigen, wird die Farbe des nach der Reduktion erhaltenen Glanzpigments durch die Absorptionsfarbe der reduzierten Titanspezies (niedere Titanoxide wie Ti₃O₅, Ti₂O₃ bis zu TiO, Titanoxynitride sowie Titannitrid) vorgegeben. Diese Pigmente sind daher, wie auch die durch Reduktion der blau reflektierenden Substratplättchen mit einer geometrischen TiO₂-Schichtdicke von etwa 120 bis 130 nm erhaltenen Glanzpigmente, intensiv blau gefärbt.

Andersfarbige, bunte Glanzpigmente, bei denen die jeweilige Interferenzfarbe der Substratplättchen verstärkt ist, werden durch unvollständige Reduktion von mit etwa 40 bis 160 nm, insbesondere 60 bis 140 nm dicken TiO₂-Schichten belegten SiO₂-Plättchen erhalten.

In diesen anreduzierten TiO₂-Schichten liegt neben den genannten reduzierten Spezies auch unverändertes Titandioxid vor. Bevorzugt beträgt der Gehalt an den reduzierten Titanverbindungen 0,1 bis 50 Gew.-%, vor allem 0,1 bis 10 Gew.-%, jeweils bezogen auf das noch enthaltende TiO₂.

Mit zunehmendem Ausmaß der Reduktion verschiebt sich auch bei diesen Substratmaterialien die Körperfarbe der Glanzpigmente in Richtung der Absorptionsfarbe der Reduktionsprodukte des Titans.

Die Reduktion der TiO₂-beschichteten Substratplättchen erfolgt vorteilhaft durch Erhitzen der Plättchen in Gegenwart reduzierend wirkender Gase.

Als reduzierende Gase eignen sich dabei insbesondere Ammoniakgas, Wasserstoff und flüchtige Kohlenwasserstoffe sowie auch Gemische von Ammoniakgas mit Wasserstoff oder Kohlenwasserstoffen, die bevorzugt im Gemisch mit Inertgasen wie Stickstoff eingesetzt werden (vgl. die nicht vorveröffentlichten DE-A-19511696 und 19511697 und die dort u.a. genannte EP-A-322 071).

Bevorzugte reduzierende Gase sind Ammoniakgas und Gemische von Ammoniakgas mit flüchtigen Kohlenwasserstoffen, die vorzugsweise mit Stickstoff verdünnt werden. Bei der Verwendung von Ammoniakgas allein sind Stickstoffanteile an der Gesamtgasmenge von bis zu 90 Vol.-% und bei der Verwendung der Gasgemische von 10 bis 60 Vol.-% empfehlenswert.

Für die Gasgemische sind vor allem gesättigte C₁-C₈-, besonders C₁-C₄-Kohlenwasserstoffe, ganz besonders Methan, Ethan und Propan geeignet. Ein Volumenverhältnis Ammoniak : Kohlenwasserstoff (C₁-C₃-Alkan) von 4:1 bis 1:4, insbesondere 3:2 bis 2:3, ist dabei bevorzugt.

Bei der Reduktion mit Ammoniak/Kohlenwasserstoff-Gasgemischen werden reduzierte Glanzpigmente erhalten, deren TiO₂-Schicht zusätzlich Kohlenstoff enthält und die sich, insbesondere wenn die Reduktion bei Temperaturen <800°C vorgenommen wurde, durch hervorragende Echtheiten (Schwitzwasserbeständigkeit, Wetterechtheit und damit auch Lichtechtheit) auszeichnen.

Mit zunehmendem Kohlenstoffgehalt werden immer dunklere, schließlich schwarze Glanzpigmente gebildet. Zur Herstellung farbiger Glanzpigmente ist daher ein niedriger Kohlenstoffgehalt von etwa 0,2 bis 10 Gew.-%. bevorzugt 0,2 bis 5 Gew.-%, jeweils bezogen auf das Gewicht des Pigments, besonders geeignet. Dieser Kohlenstoffgehalt kann durch Steuerung der Reduktionsbedingungen, z.B. durch einen Anteil des Kohlenwasserstoffgases am Gesamtvolumen der reduzierenden Gase von <50 Vol.-%, durch Verwendung niederer Alkane, durch kurze Reduktionszeiten (etwa 1 bis 2 h) und durch höhere Reduktionstemperaturen (etwa 750 bis 900°C), gezielt eingestellt werden.

Üblicherweise liegen die Reduktionstemperaturen bei 500 bis 1000°C, bevorzugt 600 bis 900°C und besonders bevorzugt 700 bis 850°C.

Die Reduktion kann erfindungsgemäß sowohl kontinuierlich, z.B. in einem beheizten, inertisierten Drehrohrofen, in den das Glimmerpigment eingetragen und die reduzierenden Gase im Gemisch mit einem Inertgas eingeleitet werden, als auch diskontinuierlich, z.B. in einer beheizten, inertisierten Drehtrommel mit Gaszu- und -ableitung oder dem oben beschriebenen Wirbelbettreaktor, durchgeführt werden, wobei zweckmäßigerweise für eine allseitige Umspülung der Glimmerplättchen mit dem Reduktionsgas gesorgt wird, was im Drehrohrofen oder in der Drehtrommel vorzugsweise durch Stolperleisten ermöglicht wird.

Nach Beendigung der Reduktion, die im allgemeinen 1 bis 10 h, besonders 2 bis 7 h, dauert, wird das Glanzpigment vorzugsweise unter Inertgas abgekühlt. Gewünschtenfalls kann ein Desagglomerierungsschritt, beispielsweise in einem mit Schlagmessern ausgerüsteten Mischer, angeschlossen werden.

Die erfindungsgemäßen Glanzpigmente zeichnen sich durch vorteilhafte Anwendungseigenschaften (hohe Echtheiten insbesondere der mit Ammoniak/Kohlenwasserstoff-Gasgemischen reduzierten Pigmente) und vor allem auch gute koloristische Eigenschaften (gegenüber analog beschichteten Glimmerpigmenten deutlich kräftigeren Interferenzfarben) aus.

Sie eignen sich vorteilhaft für viele Zwecke, wie zur Einfärbung von Kunststoffen, Gläsern, keramischen Produkten, Zubereitungen der dekorativen Kosmetik und besonders von Lacken, insbesondere auch Automobillacken. Tinten und Druckfarben, vor allem Sicherheitsdruckfarben. Bei der Applikation im Druck sind alle industrieüblichen Druckverfahren, z.B. Siebdruck, Tiefdruck, Bronzierdruck, Flexodruck und Offsetdruck, geeignet.

Für diese Anwendungszwecke lassen sich die erfindungsgemäßen Pigmente auch vorteilhaft in Abmischung mit transparenten und deckenden Weiß-, Bunt- und Schwarzpigmenten sowie auch herkömmlichen transparenten, bunten und schwarzen Glanzpigmenten auf der Basis von metalloxidbeschichteten Glimmer- und Metallpigmenten, plättchenförmigen Eisenoxiden, Graphit, Molybdänsulfid und plättchenförmigen organischen Pigmenten verwenden.

## Patentansprüche

1. Glanzpigmente auf Basis beschichteter Siliciumdioxidplättchen, erhältlich durch Belegung der Siliciumdioxidplättchen mit einer im wesentlichen aus Titandioxid bestehenden Schicht und anschließendes Erhitzen der belegten Plättchen in einer reduzierenden Atmosphäre.

2. Glanzpigmente nach Anspruch 1, bei denen die Titandioxidschicht durch Gasphasenzersetzung flüchtiger Titanverbindungen in Gegenwart von Wasserdampf oder naßchemisch durch Hydrolyse von Titanverbindungen auf die Siliciumdioxidplättchen aufgebracht wird.

3. Glanzpigmente nach Anspruch 1 oder 2, bei denen die mit Titandioxid belegten Siliciumdioxidplättchen in Gegenwart von Ammoniakgas, Wasserstoff, flüchtigen Kohlenwasserstoffen, Gemischen von Ammoniakgas und Wasserstoff oder Gemischen von Ammoniakgas und flüchtigen Kohlenwasserstoffen erhitzt werden.

4. Glanzpigmente nach den Ansprüchen 1 bis 3, bei denen die mit Titandioxid belegten Siliciumdioxidplättchen auf 500 bis 1000°C erhitzt werden.

5. Verfahren zur Herstellung von Glanzpigmenten gemäß den Verfahrensschritten von Anspruch 1.

6. Verwendung von Glanzpigmenten gemäß den Ansprüchen 1 bis 4 zur Einfärbung von Lacken, Druckfarben, Tinten, Kunststoffen, Gläsern, keramischen Produkten und Zubereitungen der dekorativen Kosmetik.

## Claims

1. Luster pigments based on coated silicon dioxide platelets, obtainable by coating the silicon dioxide platelets with a layer consisting essentially of titania and subsequently heating the coated platelets in a reducing atmosphere.

2. Luster pigments as claimed in claim 1, wherefor the titania layer is applied to the silicon dioxide platelets by gas phase decomposition of volatile titanium compounds in the presence of water vapor or wet-chemically by hydrolysis of titanium compounds.

3. Luster pigments as claimed in claim 1 or 2, wherefor the titania-coated silicon dioxide platelets are heated in the presence of ammonia gas, hydrogen, volatile hydrocarbons, mixtures of ammonia gas and hydrogen or mixtures of ammonia gas and volatile hydrocarbons.

4. Luster pigments as claimed in any of claims 1 to 3, wherefor the titania-coated silicon dioxide platelets are heated to 500-1000°C.

5. A process for preparing luster pigments by the steps of claim 1.

6. The use of luster pigments as claimed in any of claims 1 to 4, for coloring paints, inks, including printing inks, plastics, glasses, ceramic products and decorative cosmetic preparations.

## Revendications

1. Pigments brillants à base de plaquettes de dioxyde de silicium revêtues, que l'on peut obtenir en recouvrant des plaquettes de dioxyde de silicium avec une couche composée essentiellement de dioxyde de titane et en chauffant ensuite les plaquettes recouvertes dans une atmosphère réductrice.

2. Pigments brillants suivant la revendication 1, dans lesquels la couche de dioxyde de titane est déposée sur les plaquettes de dioxyde de silicium par dissociation en phase gazeuse de composés de titane volatils en présence de vapeur d'eau ou par voie chimique humide par hydrolyse de composés de titane.

3. Pigments brillants suivant la revendication 1 ou 2, dans lesquels les plaquettes de dioxyde de silicium recouvertes de dioxyde de titane sont chauffées en présence de gaz ammoniac, d'hydrogène, d'hydrocarbures volatils, de mélanges de gaz ammoniac et d'hydrogène ou de mélanges de gaz ammoniac et d'hydrocarbures volatils.

4. Pigments brillants suivant les revendications 1 à 3, dans lesquels les plaquettes de dioxyde de silicium recouvertes de dioxyde de titane sont chauffées à 500 à 1000°C.

5. Procédé de production de pigments brillants suivant les étapes de procédé de la revendication 1.

6. Utilisation de pigments brillants suivant les revendications 1 à 4 pour la coloration de laques, encres d'imprimerie, encres, matières plastiques, verres, produits céramiques et pour des préparations de cosmétique décorative.
